Europäisches Patentamt

(19) European Patent Office

Office européen des brevets

(11) Veröffentlichungsnummer: **0 021 021**
**B1**

(12) **EUROPÄISCHE PATENTSCHRIFT**

(45) Veröffentlichungstag der Patentschrift:
10.02.82

(51) Int. Cl.³: **B 01 D 3/00**, C 07 C 7/04

(21) Anmeldenummer: 80102743.4

(22) Anmeldetag: 17.05.80

(54) Vorrichtung zur Rückgewinnung von Xylol und/oder Paraffin und ähnlichen Substanzen für histologische Zwecke.

(30) Priorität: 26.05.79 DE 2921488

(43) Veröffentlichungstag der Anmeldung:
07.01.81 Patentblatt 81/1

(45) Bekanntmachung des Hinweises auf die Patenterteilung:
10.02.82 Patentblatt 82/6

(84) Benannte Vertragsstaaten:
CH FR GB IT LI NL SE

(56) Entgegenhaltungen:
DE-B-1 118 753
DE-B-1 119 229
DE-C-1 238 887
FR-A1-2 284 363
FR-A2-2 405 079
Kirschbaum «Destiller- und
Rektifiziertechnik» Springer
Verlag 1960, 3. Auflage, S. 94

(73) Patentinhaber: Hamann, Wolfgang, Dr.,
Felsenkellerweg 3, D-6333 Braunfels (DE)

(72) Erfinder: Hamann, Wolfgang, Dr., Felsenkellerweg 3,
D-6333 Braunfels (DE)

(74) Vertreter: Knefel, Siegfried, Dipl.-Math.,
Wertherstrasse 16 Postfach 1924, D-6330 Wetzlar (DE)

Vorrichtung zur Rückgewinnung von Xylol und/oder Paraffin und ähnlichen Substanzen für histologische Zwecke

Die Erfindung betrifft eine Vorrichtung zur Rückgewinnung von Xylol und/oder Paraffin für histologische Zwecke in der Pathologie nach dem Gattungsbegriff des Anspruches 1.

In der Pathologie wird als Lösungsmittel Xylol verwandt, das nach dem Gebrauch von Wasser, Alkohol, Gewebeteilchen und sonstigen Feststoffen verunreinigt ist. Andererseits werden solche Gewebeteilchen in Paraffin eingebettet, um Mikroschnitte herstellen zu können.

Will man das Xylol zurückgewinnen, kann man das verunreinigte Xylol destillieren, beispielsweise in Form einer fraktionierten Destillation. Eine solche Destillation ist aufwendig und kann nur mit grossem Zeitaufwand durchgeführt werden. Nach dem Stand der Technik werden hierfür sogenannte Rotationsverdampfer verwendet, welche vornehmlich aus Glas bestehen. Ihre Handhabung erfordert Fachkenntnissse. Darüber hinaus lassen sich diese Geräte nur sehr schwer von den Rückständen reinigen, weil sie leicht zu Bruch gehen. Unter dieser Voraussetzung wird Xylol nur in geringem Umfang und Paraffin überhaupt nicht in den medizinischen Labors zurückgewonnen.

Aufgabe der Erfindung ist es, eine Vorrichtung anzugeben, mit der das Xylol auch von angelernten Hilfskräften und darüber hinaus auch in kürzester Zeit rückgewonnen werden kann, wobei Glasbruch vermieden wird und eine Reinigung ohne grossen Zeitaufwand durchführbar ist, und die es darüber hinaus gestattet, auch Paraffin zurückzugewinnen, insbesondere das Paraffin von Xylol zu trennen.

Diese Aufgabe wird durch die Vorrichtung nach den Merkmalen des Anspruches 1 gelöst.

Zwar sind Destillationsanlagen bekannt, die aus einem Verdampfungskessel, nachgeschaltetem Kühler und mehreren Auffangbehältern bestehen, (FR-A-2 284 363, Fig. 1, und Kirschbaum «Destillier- und Rektifiziertechnik» 3. Auflage, Springer Verlag 1960, S. 94) und auch die Steuerung eines Destillationsarbeitsganges über die Einstellung eines bestimmten Temperaturniveaus ist gebräuchlich (FR-A-2 405 079), jedoch gibt dieser Stand der Technik keinen Hinweis darauf, Xylol aus einem Gewebeteilchen enthaltenden Xylol-Wasser-Alkohol-Gemisch durch fraktionierte offene Destillation bei geregeltem Druck und geregelter Temperatur in zwei Destillationsstufen auf die beanspruchte relativ einfache Weise zurückzugewinnen.

Denn gemäss der Erfindung wird durch eine einfache Zweistufen-Regeleinstellung die Temperatur und der Druck im Innern des Verdampfungskessels derart reguliert, dass zunächst das Wasser und der Alkohol verdampfen und selbsttätig in einem besonderen Behälter aufgefangen werden. In einer zweiten Stufe verdampft das Xylol nach Umschaltung der Steuereinrichtung und wird in einem weiteren Behälter aufgefangen.

Die Rückstände sammeln sich im Verdampfungskessel an und können nach Öffnen eines Absperrhahnes ausfliessen, nötigenfalls nach Einfüllen eines Reinigungsmittels in diesen Kessel, um zum Beispiel Paraffinrückstände zu lösen. Ein Auseinandernehmen der Anlage zum Zwecke der Reinigung ist nicht mehr erforderlich.

Der Kondensator selbst wird gemäss der Erfindung gleichzeitig auch zur Trennung des Xylols vom Paraffin verwendet, indem das Paraffin in fester Form, beispielsweise in einen Trichter für den Verdampfungsbehälter gefüllt wird, der etwa auf 60 bis 70 °C aufheizbar ist, so dass das Paraffin schmilzt. Ein Papierfilter hält Gewebeteilchen und sonstige Rückstände fest. Der Verdampfungsbehälter wird derart aufgeheizt, dass das Xylol verdampft, und der Druck wird geeignet reguliert. Dieser Behälter steht ebenfalls mit dem Kondensator der Gesamtanlage in Verbindung, so dass das Xylol kondensiert und in den dafür ohnehin vorgesehenen Behälter fliesst.

Aus den Unteransprüchen und der Beschreibung eines Ausführungsbeispiels sind weitere Einzelheiten der Erfindung zu entnehmen.

Auf der Zeichnung ist ein Ausführungsbeispiel der Erfindung dargestellt, und zwar zeigen:

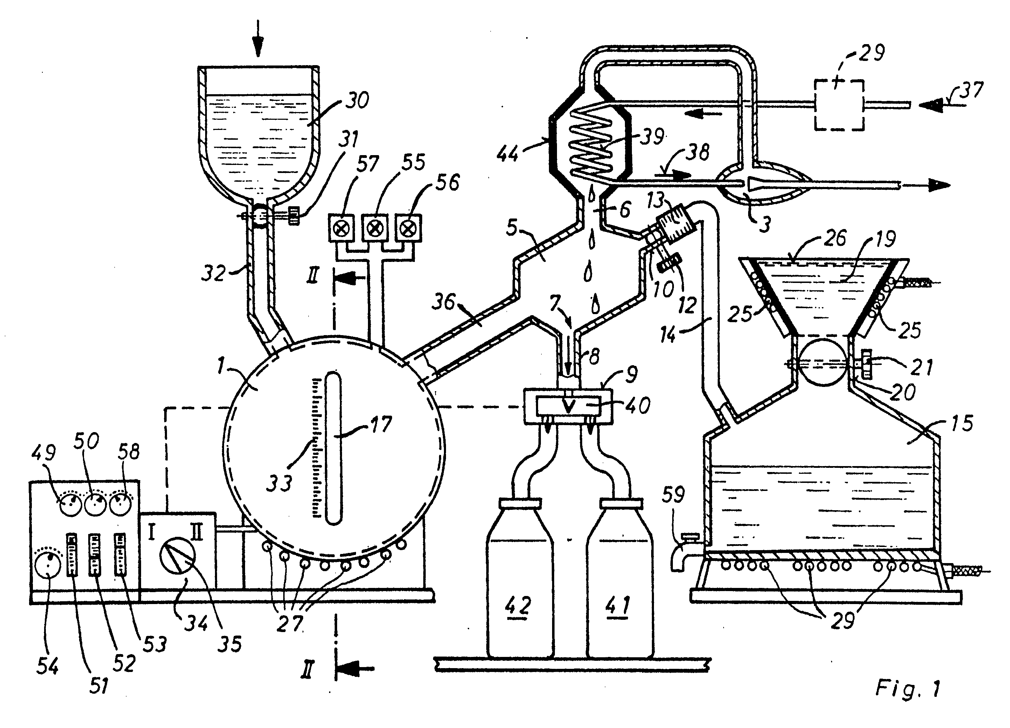

Fig. 1 die schematische Darstellung der Anlage;

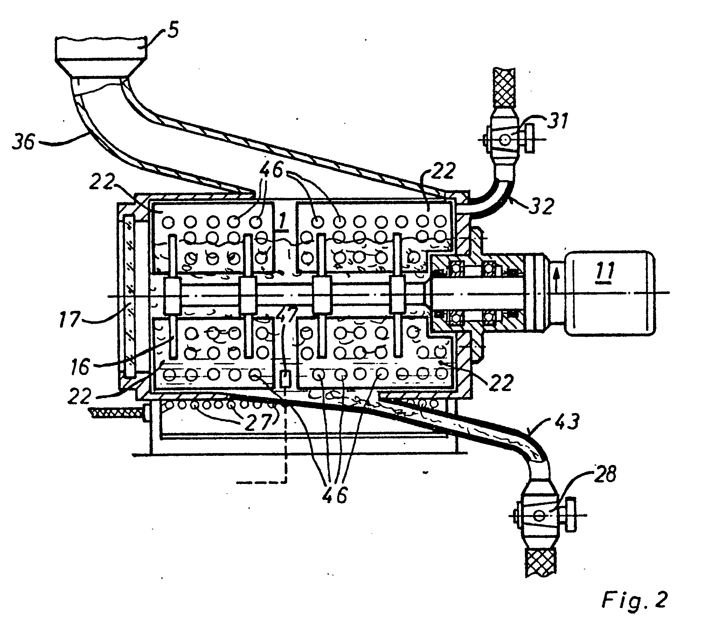

Fig. 2 einen Schnitt nach der Linie II-II der Fig. 1.

In einen trichterartigen Behälter 30 wird das verunreinigte Xylol eingefüllt. Mit Hilfe eines Absperrhahnes 31 kann über eine Leitung 32 das verunreinigte Xylol in den Verdampfungskessel 1 gefüllt werden, gegebenenfalls unter Zwischenschaltung eines Papierfilters oder dergleichen. Der Kessel 1 wird vorteilhaft etwa zur Hälfte gefüllt. Der Füllungsgrad ist durch ein Schauglas 17 beobachtbar. Das Schauglas kann eine Skala 33 tragen. Um den Kessel 1 herum oder zumindest an seiner Unterseite sind Heizplatten oder Heizspiralen 27 vorgesehen, mit deren Hilfe der Kesselinhalt erhitzt wird. Ein durch einen Motor 11 betriebenes Rührwerk 16 wälzt das im Kessel befindliche Xylol ständig um. Das Rührwerk besitzt Schaufeln 22 mit Bohrungen 46. Die Schaufelränder reichen bis in die unmittelbare Nähe der inneren Kesselwandung, so dass der Kesselinhalt mit der gesamten Kesselwandung in Berührung kommt. Hierdurch wird der Wärmeübergang auf den Kesselinhalt, insbesondere wenn der Kessel rundherum beheizt wird, vergrössert und damit der Siedevorgang intensiviert. Gleichzeitig wird ein Siedeverzug verhindert.

Mit Hilfe einer elektrischen Steueranlage 34 werden die Temperatur und gegebenenfalls der Druck im Kessel 1 so reguliert, dass beispielsweise durch Einstellen eines Betätigungsknopfes 35 auf die Stellung I im Kessel eine vorgewählte Temperatur von etwa 90 °C und ein Druck von 0,666 bar herrschen. Die Temperatur kann hierzu mit Hilfe eines Drehknopfes 49 bei Erstinbetriebnahme des Kessels eingestellt werden und der gewünschte Druck mit Hilfe einer Einstellskala 51 reguliert werden. Die jeweils im Kessel herrschende Temperatur wird mit Hilfe eines Temperaturfühlers 47 auf einer Skala 54 angezeigt.

Die Schaltanlage weist ferner Thermostate auf (nicht dargestellt), welche die eingestellte Temperatur im Kessel halten. Der eingestellte Druck wird mittels eines Regelventiles 55 konstant gehalten.

Bei der genannten Einstellung verdampfen der im Xylol enthaltene Alkohol und das Wasser. Über ein Rohr 36 mit einem Dampfentspannungsgefäss 5 werden die Wasser- und Alkoholdämpfe einem Kondensator 44 zugeführt. Die Kühlung der Dämpfe erfolgt mit Hilfe von Wasser, das einer üblichen Wasserleitung entnommen werden kann, und das in Richtung der Pfeile 37 und 38 durch Rohrschlangen 39 des Kondensators 44 fliesst. Zur Kühlung kann aber auch ein besonderes Kühlmittel verwendet werden, das mit Hilfe eines Aggregates 29 vorgekühlt wird.

Das Kondensat läuft über den Rohrstutzen 6 zurück in das Dampfentspannungsgefäss 5. Das Dampfentspannungsgefäss 5 ist so ausgebildet, dass sich das Kondensat an einer trichterförmigen Öffnung 7 sammelt und dann durch ein Verbindungsstück 8 zu einem Verteiler 9 fliesst. Der Verteiler 9 enthält ein Magnetventil 40, über das das Kondensat in einen Behälter 41 fliesst, der das Wasser und den Alkohol auffängt. Das Magnetventil wird vom Drehknopf 35 aus elektrisch gesteuert. Das Verbindungsstück 8 oder der Kondensator selbst enthalten ein Schauglas (nicht dargestellt) oder bestehen aus Glas, um die Kondensatbildung beobachten zu können.

Sind das Wasser und der Alkohol verdampft, werden durch Drehen des Schaltknopfes 35 in die Stellung II die Temperatur erhöht und der Druck im Kessel 1 vermindert, so dass beispielsweise eine mit Hilfe eines Drehknopfes 50 eingestellte Temperatur von etwa 110 °C erhalten wird und der Druck auf 0,266 bar abfällt. Der Druck in der Stellung II des Schaltknopfes 35 kann auf einer Skala 52 eingestellt werden. Die Druckskala 52 arbeitet elektrisch mit einem Regelventil 56 zusammen.

Unter diesen Verhältnissen verdampft das Xylol. Die Dämpfe gelangen über das Rohr 36 und das Druckausgleichsgefäss 5 wiederum in den Kondensator 44. Das Magnetventil 40 nimmt bei Betätigung des Drehknopfes 35 eine derartige Stellung ein, dass das Destillat jetzt in einen Behälter 42 läuft, was wiederum beobachtbar ist.

Die Temperatur und der Druck können entsprechend den Verdampfungskurven der zu verdampfenden Medien ein für allemal vorgewählt werden.

Das Wasser der Kühlschlange 39 strömt durch eine Wasserstrahlpumpe 3, welche stets danach trachtet, in der Anlage einen Unterdruck zu erzeugen. Dementsprechend sind die Regelventile 55 und 56 so ausgelegt, dass bei Entstehen eines zu grossen Unterdruckes Aussenluft in den Kessel und in den Kondensator nachströmt.

Aus Sicherheitsgründen ist ein weiteres Ventil 57 vorgesehen, das als Überdruckventil ausgelegt ist, das sich beispielsweise bei Erreichen einer Temperatur von 130 °C und dem damit entstehenden Überdruck automatisch öffnet. Die Temperatur kann mit Hilfe eines Drehknopfes 58 eingestellt werden und der maximale Überdruck mit Hilfe der Skala 53.

Im Kessel 1 verbleiben schliesslich Reststoffe, wie Gewebeteilchen und dergleichen mehr. Diese setzen sich am Boden ab. Mit Hilfe eines Rohres 43 und eines Absperrhahnes 28 können diese Reststoffe abgelassen werden, und ein erneuter Destillationsvorgang kann beginnen.

Das Dampfentspannungsgefäss 5 läuft in eine Verlängerung 10 aus, welche mit Hilfe eines Absperrhahnes 12 verschliessbar ist. Mit Hilfe einer Muffe 13 ist die Verlängerung mit einem Rohr 14 verbindbar, das in einen Behälter 15 mündet. Der Behälter 15 läuft in einen Trichter 19 aus, dessen Hals 20 durch einen Absperrhahn 21 verschliessbar ist. Um den Trichter herum liegt eine Heizspirale 25, welche das Füllgut auf etwa 60° bis 70 °C erhitzt. Ausserdem ist in den Trichter ein mehr oder minder grobes Filter, zum Beispiel ein Papierfilter 26, eingelegt. Der Behälter 5 ist mittels Heizspiralen 29 aufheizbar, beispielsweise bis zu einer Temperatur von 200 °C.

In den Trichter wird das mit Xylol verunreinigte Paraffin in fester Form geschüttet. Das Paraffin schmilzt aufgrund der Heizwirkung der Heizspirale 23 und fliesst zusammen mit dem Xylol in den Behälter 15. Grobe Rückstände, wie Gewebeteilchen und dergleichen, werden im Papierfilter 26 zurückgehalten. Im Behälter 15 verdampft das Xylol bei etwa 110 °C und bei einem von der Wasserpumpe 3 erzeugten Druck von 0,266 bar. Die Xyloldämpfe gelangen in den Kondensator 44. Das sich an der Spirale 39 bildende Kondensat läuft wiederum über das Rohr 8 und den Verteiler 9 in den Auffangbehälter 42.

Für die Verdampfung des Xylols im Behälter 15 gelten dieselben Temperatur- und Druckverhältnisse wie bei der Verdampfung im Kessel 1.

Im Behälter 15 bleibt gereinigtes Paraffin zurück, das ebenso wie das Xylol erneut verwendet werden kann. Mit Hilfe eines Absperrhahnes 59 kann das Paraffin abgelassen werden. Verunreinigungen in fester Form werden im Filter 26 zurückgehalten, das bei Bedarf ausgewechselt werden kann.

Nachdem die Anlage einmal eingestellt worden ist, bedarf es zur Rückgewinnung des Xylols und des Paraffins lediglich einer geeigneten Umschaltung des Drehknopfes 1 auf die Stellungen I und II.

Der Hauptvorteil der erfindungsgemässen Vorrichtung wird darin gesehen, dass eine robuste und äusserst einfach zu bedienende Anlage angegeben worden ist, die es gestattet, wahlweise Xylol und Paraffin zurückzugewinnen, wobei die Verunreinigungen in Form von festen Gewebeteilchen und dergleichen entweder im Verdampfungskessel 1 zurückbleiben oder im Filter des Verdampfungsbehälters 15, in jedem Fall jedoch leicht entfernbar sind, so dass der Reinigungsaufwand gering bleibt. Hierdurch wird nicht nur ein wirtschaftlicher Laborbetrieb gesichert, sondern auch eine erhebliche Einsparung an Rohstoffen erhalten.

**Patentansprüche**

1. Vorrichtung zur Rückgewinnung von reinem Xylol aus mit Wasser, Alkohol, Gewebeteilchen und sonstigen Stoffen verunreinigtem Xylol und/oder von reinem Paraffin und ähnlichen Substanzen für histologische Zwecke aus mit Xylol und Feststoffen verunreinigtem Paraffin mit Hilfe eines an sich bekannten Verdampfungskessels mit nachgeschaltetem Kühler und mehreren Auffangbehältern, bestehend aus dem Verdampfungskessel (1) für das verunreinigte Xylol sowie dem nachgeschalteten Kühler (44) und einer Steuereinrichtung (34), welche wahlweise den Druck und die Temperatur im Verdampfungskessel (1) regelt und gleichzeitig auf einen dem Kühler (44) nachgeschalteten Verteiler (9) mit einem Steuerventil (40) wirkt, das wahlweise den Kondensatabfluss mit einem, vorzugsweise zwei der nachgeschalteten Auffangbehälter (41, 42) verbindet, wobei der Kühler (44) wahlweise mit einem, der Aufnahme des verunreinigten Paraffins dienenden Verdampfungsbehälter (15), der mit einem heizbaren Trichter (19) mit Filter (26) zum Aufschmelzen des festen, verunreinigten Paraffins ausgerüstet ist, verbindbar ist und die Temperatur und der Druck in diesem Behälter (15) ebenfalls regelbar sind.

2. Vorrichtung nach Anspruch 1, dadurch gekennzeichnet, dass der Verdampfungskessel (1) ein Schauglas (17) aufweist.

3. Vorrichtung nach Anspruch 1, dadurch gekennzeichnet, dass für die Erzeugung eines Unterdruckes im Kessel (1) und im Behälter (15) sowie im Kühler (44) eine Vakuumpumpe vorgesehen ist.

4. Vorrichtung nach Anspruch 1, gekennzeichnet durch eine Druckregeleinrichtung sowie dadurch, dass die Druckregeleinrichtung durch wenigstens ein Regelventil gebildet ist, das das Innere der Anlage gegebenenfalls mit der Aussenluft verbindet.

5. Vorrichtung nach Anspruch 3, dadurch gekennzeichnet, dass die Vakuumpumpe eine Wasserstrahlpumpe (3) ist, die von durch eine Kühlschlange (39) des Kühlers (44) fliessendem Kühlwasser gespeist wird.

6. Vorrichtung nach Anspruch 1, dadurch gekennzeichnet, dass durch die Kühlschlange des Kühlers eine Kühlflüssigkeit fliesst und der Kühlschlange ein die Kühlflüssigkeit abkühlendes Aggregat (29) vorgelagert ist oder dass die Kühlschlange (39) mit dem Wasserleitungsnetz verbunden ist.

7. Vorrichtung nach Anspruch 1, dadurch gekennzeichnet, dass zwischen dem Kessel (1) und dem Kühler (44) ein Dampfentspannungsgefäss (5) vorgesehen ist, durch das die Dämpfe einerseits in den Kühler (44) gelangen und durch das andererseits das Kondensat zurückfliesst, um von hier aus zu dem nachgeschalteten Verteiler (9) zu gelangen, und dass das Dampfentspannungsgefäss (5) mit dem Verdampfungsbehälter (15) wahlweise verbindbar ist.

8. Vorrichtung nach Anspruch 1, dadurch gekennzeichnet, dass der Kühler (44) und/oder das Dampfentspannungsgefäss (5) wenigstens teilweise transparent ausgebildet ist.

9. Vorrichtung nach Anspruch 1, dadurch gekennzeichnet, dass der Kessel (1) am Boden einen wahlweise verschliessbaren Ablauf (Rohr (43)) für die Rückstände aufweist und der Verdampfungsbehälter (15) einen Ablasshahn (59) aufweist.

10. Vorrichtung nach Anspruch 1, dadurch gekennzeichnet, dass in den Trichter (19) und/oder einen Behälter für das verunreinigte Xylol (30) ein Papierfilter (26) oder dergleichen einlegbar ist.

11. Vorrichtung nach Anspruch 1, dadurch gekennzeichnet, dass der Kessel (1) und/oder der Verdampfungsbehälter (15) ein Rührwerk (16) aufweist, dessen Schaufeln (22) bis an die innere Kessel- und/oder Behälterwandung reichen.

12. Vorrichtung nach Anspruch 1, gekennzeichnet durch einen Zweistufenschalter (35), der in der einen Stufe den Druck und die Temperatur der Anlage zur Verdampfung des Wassers und des Alkohols in Verbindung mit dem Regelventil (55) steuert sowie das Magnetventil (40) geeignet schaltet und in der anderen Stufe den Druck und die Temperatur für die Verdampfung des Xylols in Verbindung mit einem Regelventil (56) steuert und gleichzeitig das Magnetventil umschaltet, und dass zusätzlich ein Überdruckventil vorgesehen ist.

13. Vorrichtung nach Anspruch 12, dadurch gekennzeichnet, dass zwischen dem Verdampfungsbehälter (15) und dem Dampfentspannungsgefäss (5) ein Absperrhahn (12) vorgesehen ist und zwischen dem Verdampfungskessel (1) und dem Dampfentspannungsgefäss (5) ein weiterer Absperrhahn oder ein Ventil vorgesehen ist.

**Claims**

1. Appliance for the regeneration of pure Xylene out of Xylene which is soiled by water, tissue-parts and other substances and/or of pure paraffin and similar substances for histological purposes out of paraffin, which is soiled by Xylene and solid matters with the help of an evaporation kettle with a tandemarranged cooler with several collecting containers which is already known as such consisting of the evaporation kettle (1) for the soiled Xylene and the tandemarranged cooler (44) and of a control system (34) which alternatively adjusts pressure and temperature in the evaporation kettle (1) and at the same time influences a distributer (9) with a control valve (40) which is tandemarranged to the cooler (44) and which alternatively connects the condensation running off with one, preferably two collecting vessels (41, 42) whereby the cooler (44) can be connected alternatively with an evaporation vessel (15) for the collection of soiled paraffin which is supplied with a heatable funnel (19) with a filter (26) to liquefy the solid and soiled paraffin and whereby pressure and temperature in this vessel (15) are also adjustable.

2. Appliance corresponding to claim No. 1, characterized by an inspection glass (17) in the evaporation kettle.

3. Appliance corresponding to claim No. 1, characterized by a vacuum pump to produce underpressure in the kettle (1) and in the vessel (15) as well as in the cooler (44).

4. Appliance corresponding to claim No. 1, characterized by a pressure regulating device, consisting of at least one regulating valve, which connects the inside of the unit with the outside air if necessary.

5. Appliance corresponding to claim No. 3, characterized by the fact, that the vacuum pump is a water-jet pump (3), which is fed by the cooling water which flows through a cooling soil (39) of the cooler (44).

6. Appliance corresponding to claim No. 1, characterized by the fact, that a cooling fluid flows through the cooling soil of the cooler and an aggregate to cool off the cooling (39) is established in front of the cooling soil or that the cooling soil (39) is connected to the water system.

7. Appliance corresponding to claim No. 1., characterized by a vessel for the let-down of steam (5), which is designated between the kettle (1) and the cooler (44) and through which on the one hand the steam gets back into the cooler (44), on the other hand the condensate can flow back to reach the tandemarranged distributor (9) and which alternatively connects the vessel for the let-down of steam (5) with the evaporation vessel (15).

8. Appliance corresponding to claim No. 1 by the fact that the cooler (44) and/or the vessel for the let-down of steam (5) are at least partially built of transparent material.

9. Appliance corresponding to claim No. 1, characterized by the fact that on the bottom of the kettle (1) there is a lockable discharge (tube (43)) for the remainder and that the evaporation vessel (15) has a discharge cock (59).

10. Appliance corresponding to claim No. 1, characterized by the fact that a paper filter (29) or the like can be inserted in the funnel (19) and/or in a vessel for the soiled Xylene (30).

11. Appliance corresponding to claim No. 1, characterized by the fact that the kettle (1) and/or the evaporation vessel (15) has an agitator (16) whose shovels (22) reach to the inner wall of the kettle and/or to the inner wall of the vessel.

12. Appliance corresponding to claim No. 1, characterized by a two-stage switch (35), which on one stage controls pressure and temperature for the evaporation of water and alcohol in connection with a control valve (55) and switches the solenoid valve (40) suitable as well as on the other stage controls pressure and temperature of the Xylene in connection with a control valve (56), and at the same time switches over the solenoid valve, and that an excess-pressure valve is designated in addition to this.

13. Appliance corresponding to claim No. 1, characterized by the fact that a stop-cock (12) is designated between the evaporation vessel (15) and the vessel for the let-down of steam (5) and that between the evaporation-kettle (1) and the vessel for the let-down of steam (5) there is designated a further stop-cock or valve.

**Revendications**

1. Dispositif de récupération de xylène pur à partir de xylène souillé d'eau, d'alcool, de particules de tissus et d'autres matières et/ou de paraffine pure et substances analogues, pour études des tissus à des fins histologiques, à partir de paraffine souillée de xylène et de matières solides, au moyen d'un évaporateur généralement connu et de plusieurs récipients collecteurs, se composant d'une cuve de vaporisation (1) pour le xylène souillé, ainsi que d'un réfrigérant intercalé en arrière (44) et d'un dispositif de commande (34), qui régule au choix la pression et la température dans la cuve de vaporisation (1) et en même temps agit au moyen d'une valve de régulation (40) sur un répartiteur (9) raccordé à la suite du réfrigérant (44); celle-ci dirige, au choix, l'écoulement du condensat dans un, de préférence dans deux récipients collecteurs (41, 42), qui sont intercalés, le réfrigérant (44) étant reliable, si on le désire, à un autre récipient de vaporisation (15) servant à la réception de la paraffine souillée, ce récipient est équipé d'un entonnoir chauffant (19) avec un filtre (26) pour la fusion de la paraffine solide, non pure, et la température et la pression sont également réglables dans ce récipient (15).

2. Dispositif selon la revendication 1, prévoyant la présence d'une fenêtre (17) dans la cuve de vaporisation.

3. Dispositif découlant de la revendication 1, prévoit une pompe à vide permettant la production d'une dépression dans la cuve (1) le récipient (15) ainsi que dans le réfrigérant (44).

4. Dispositif selon la revendication 1, prévoit un système de réglage de pression, qui soit constitué d'au moins d'une soupape de régulation, qui relie l'intérieur de l'installation avec l'air ambiant, le cas échéant.

5. Dispositif selon la revendication 3, prévoit une pompe à vide, qui soit une pompe par jet d'eau (3) alimentée d'eau froide circulant à travers le serpentin refroidisseur (39) du réfrigérant (44).

6. Dispositif selon la revendication 1, prévoit un liquide de refroidissement coulant à travers le serpentin refroidisseur du réfrigérant, et par l'interposition, en amont du serpentin, d'un agrégat (29) refroidissant le liquide de refroidissement ou bien le raccordement du serpentin (39) à une conduite d'eau courante.

7. Dispositif selon la revendication 1, prévoit un récipient de vapeur détendeur (5) entre la cuve (1) et le réfrigérant (44), afin que d'une part la vapeur parvienne dans le réfrigérant (44) et que d'autre part le condensat reflue et gagne le répartiteur (9); il prévoit aussi que le détendeur (5) soit éventuellement reliable avec le récipient de vaporisation (15).

8. Dispositif selon la revendication 1, prévoit que le réfrigérant (44) et/ou le détendeur (5) soient, au moins en partie, transparents.

9. Dispositif selon la revendication 1, prévoit la présence au fond de la cuve (1) d'un écoulement (tuyau (43)) pour les résidus, fermable à volonté, et pour le récipient de vaporisation (15) celle d'un robinet de vidange (59).

10. Dispositif selon la revendication 1, prévoit l'adaptation d'un papier filtre (26) ou analogue, dans l'entonnoir (19) et/ou dans un récipient (30) prévu pour le xylène souillé.

11. Dispositif selon la revendication 1, prévoit un agitateur (16) dans la cuve (1) et/ou le récipient de vaporisation (15), dont les pelles (22) atteignent l'intérieur de la cloison de la cuve et/ou du récipient.

12. Dispositif selon la revendication 1, prévoit un commutateur à 2 positions (35); en position I, il commande la pression et la température de l'installation pour l'évaporation de l'eau et de l'alcool en liaison avec la soupape de régulation (55), puis ce commutateur enclenche la vanne magnétique (40) au moment désiré; en position II, le commutateur régule la pression et la température d'évaporation du xylène, en liaison avec une soupape de réglage (56) et en même temps inverse la vanne magnétique, et en plus, il est prévu une soupape de surpression.

13. Dispositif selon la revendication 12, qui prévoit un robinet d'arrêt (12) entre le récipient de vaporisation et le récipient détendeur de vapeur (5), et un autre robinet d'arrêt ou une soupape entre la cuve (1) et le détendeur (5).

Fig. 1

Fig. 2